(19) **Europäisches Patentamt**

**European Patent Office**

**Office européen des brevets**

(11) **EP 0 699 476 B1**

(12) **EUROPÄISCHE PATENTSCHRIFT**

(45) Veröffentlichungstag und Bekanntmachung des Hinweises auf die Patenterteilung:
04.11.1998 Patentblatt 1998/45

(51) Int. Cl.$^6$: **B01J 23/22**, B01J 23/18

(21) Anmeldenummer: 95112199.5

(22) Anmeldetag: 03.08.1995

(54) **Für die Ammonoxidation geeignete Trägerkatalysatoren**

Supported catalysts suitable for ammoxidation

Catalyseurs sur supports appropriés à l'ammoxydation

(84) Benannte Vertragsstaaten:
**BE DE FR GB NL**

(30) Priorität: **12.08.1994 DE 4428595**

(43) Veröffentlichungstag der Anmeldung:
**06.03.1996 Patentblatt 1996/10**

(73) Patentinhaber:
**BASF AKTIENGESELLSCHAFT**
**67056 Ludwigshafen (DE)**

(72) Erfinder:
• **Karrer, Lothar, Dr.**
**D-64319 Pfungstadt (DE)**
• **Pape, Frank-Friedrich, Dr.**
**D-67259 Kleinniedesheim (DE)**
• **Köhler, Ulrich, Dr.**
**D-68259 Mannheim (DE)**
• **Becker, Rainer, Dr.**
**D-67098 Bad Dürkheim (DE)**
• **Weidlich, Peter, Dr.**
**D-68159 Mannheim (DE)**
• **Hüllmann, Michael, Dr.**
**D-64625 Bensheim (DE)**
• **Kneuper, Heinz-Josef, Dr.**
**D-68163 Mannheim (DE)**

(56) Entgegenhaltungen:
EP-A- 0 222 249          EP-A- 0 352 023
EP-A- 0 389 701          EP-A- 0 558 424
US-A- 4 271 091          US-A- 4 952 389

## Beschreibung

Die vorliegende Erfindung betrifft für die Anmonoxidation geeignete Trägerkatalysatoren aus

a) einem kugelförmigen oder annähernd kugelförmigen Trägermaterial, das im wesentlichen aus Aluminiumoxid, Siliciumdioxid, Titandioxid und/oder Zirkoniumdioxid besteht und dessen Schüttdichte 0,6 bis 1,2 kg/l beträgt, und
b) einer aktiven Masse, die als wesentliche Komponenten Vanadium und Antimon in oxidischer Form enthält.

Außerdem betrifft die Erfindung ein Verfahren zur Herstellung dieser Trägerkatalysatoren sowie deren Verwendung für die Ammonoxidation iso- und heteroaromatischer Alkylverbindungen zu den entsprechenden Nitrilen.

Die Ammonoxidation von $C_1$-$C_4$-Alkyl-Iso- und Heteroaromaten wie Toluol, den Xylolen oder den Picolinen stellt ein technisch übliches Verfahren zur Synthese der entsprechenden aromatischen Nitrile dar. Die Reaktion erfolgt üblicherweise in der Gasphase unter Mitverwendung von Trägerkatalysatoren, die neben Vanadium andere Elemente wie Antimon, Chrom, Molybdän oder Phosphor in oxidischer Form enthalten. Als Träger finden hauptsächlich inerte Metalloxide wie Aluminiumoxid, Siliciumdioxid, Titanoxid oder Zirkoniumdioxid sowie Gemische dieser Oxide Verwendung.

Da man die stark exotherme Ammonoxidation technisch üblicherweise in Wirbelbettreaktoren durchführt, werden an die Katalysatoren hinsichtlich der Fluidisierbarkeit, der Abriebfestigkeit und der Fähigkeit zur Wärmeübertragung hohe Anforderungen gestellt, die bisher noch nicht befriedigend erfüllt werden konnten. Insbesondere läßt die Abriebfestigkeit dieser Katalysatoren zu wünschen übrig. Infolge des Abriebs erhält man feinen Katalysatorstaub, der aus der Wirbelzone ausgetragen wird und ausserhalb der Wirbelzone zu unerwünschten Folgereaktionen Anlaß geben kann.

Eine gute Wärmeübertragung läßt sich durch eine hohe Schüttdichte des Katalysators erzielen, der allerdings wegen der Anforderung der Fluidisierbarkeit nach oben Grenzen gesetzt sind.

Die Herstellung der Katalysatoren erfolgt derzeit nach einem seit langem bekannten Verfahren, das im Laufe der Zeit lediglich geringfügig abgewandelt wurde. So wird nach der DE-A 28 10 856 und der EP-A 222 249 Aluminiumoxid bei 900°C geglüht, zerkleinert und gesiebt. Die gewünschte Fraktion imprägniert man mit einer Lösung der aktiven Bestandteile des Katalysators, wonach man das Gemisch eindampft und die zurückgebliebene Masse trocknet, unter oxidierenden Bedingungen calciniert und anschließend zerkleinert. Ein ähnliches Verfahren ist in DE-A 16 43 630 (US-A 3 637 797) beschrieben.

Man erhält hierbei nadelförmige Teilchen, mit denen sich das Wirbelbettverfahren wegen ihrer geringen Schüttdichte von etwa 0,5 kg/l technisch nur auf aufwendige Weise beherrschen läßt. Außerdem weisen die Teilchen wegen ihrer geringen Schüttdichte eine unbefriedigende Fähigkeit zur Wärmeübertragung auf.

Der Erfindung lag daher die Aufgabe zugrunde, den genannten Nachteilen abzuhelfen.

Demgemäß wurden die eingangs definierten Trägerkatalysatoren gefunden.

Außerdem wurde ein Verfahren zur Herstellung dieser Trägerkatalysatoren sowie deren Verwendung für die Ammonoxidation gefunden.

Das kugelförmige oder annähernd kegelförmige Trägermaterial als solches ist bekannt und auch handelsüblich (im Falle des Aluminiumoxids z.B. verschiedene Typen unter der Handelsbezeichnung Puralox® der Firma Condea-Chemie, Moers). Für die Ammonoxidation wurden derartige Trägermaterialien bisher aber noch nicht eingesetzt.

Geeignete kugelförmige Teilchen haben vorzugsweise einen durchschnittlichen Formfaktor von F > 85%. Der Formfaktor sei als

$$F = (U_2)^2 / (U_1)^2$$

definiert, wobei $U_1$ den Umfang eines Teilchenquerschnitts Q und $U_2$ den Umfang eines Kreises gleicher Querschnittsfläche Q bedeutet. Die Bedingung eines Mindestformfaktors ist dann erfüllt, wenn kein Querschnitt des Teilchens einem kleineren Wert entspricht, wie sich statistisch ermitteln läßt.

Man kann das Trägermaterial herstellen, indem man die Lösung oder Suspension einer Aluminium-, Silicium, Titan- und/oder Zirkonium-Verbindung der Sprühtrocknung unterwirft und die erhaltenen Teilchen in einem Sauerstoff enthaltenden Gasstrom bei 500 bis 1200°C in die Oxide überführt. Bei der Sprühtrocknung lassen sich die gewünschte Teilchengröße und Schüttdichte in an sich bekannter Weise einstellen. Die Schüttdichte des Trägermaterials beträgt erfindungsgemäß 0,6 bis 1,2 kg/l, vorzugsweise 0,7 bis 1,0 kg/l.

Zur Herstellung der kugelförmigen Teilchen durch Sprühtrocknung von Lösungen der entsprechenden Verbindungen eignen sich z.B. Alkoholate wie Ethanolate und Isopropylate, Carboxylate wie Acetate, Sulfate und Nitrate und als suspendierte Verbindungen Hydroxide und Oxidhydrate.

Das calcinierte Trägermaterial wird dann mit einer Lösung oder Suspension von Verbindungen der aktiven Masse imprägniert, wonach die so erhaltene Masse in an sich bekannter Weise getrocknet und im Luftstrom calciniert wird. Für die Trocknung liegen die Temperaturen vorzugsweise bei 100 bis 200°C und für die Calcinierung bei 400 bis 750°C.

Zur Imprägnierung verwendet man vorzugsweise wäßrige Lösungen bzw. Suspensionen der Verbindungen der aktiven Katalysatorbestandteile, jedoch eignen sich grundsätzlich beliebige Flüssigkeiten. Vorzugsweise setzt man die Imprägnierlösung bzw. -suspension nicht in größerer Menge ein, als vom Trägermaterial aufgenommen werden kann, denn andernfalls erhält man bei der Trocknung Agglomerate, die erst wieder zerkleinert werden müssen, wobei Teilchen entstehen können, die nicht die gewünschte Kugelform haben. Man kann die Imprägnierung nach jeweiliger Zwischentrocknung auch in mehreren Schritten vornehmen.

Die Menge des Vanadiums, als Metall gerechnet, beträgt im aktiven Katalysator in der Regel 0,5 bis 10 Gew.-%, vorzugsweise 1,0 bis 6 Gew.-%, besonders 1,5 bis 5 Gew.-%, und für das Antimon, ebenfalls als Metall gerechnet, empfehlen sich Mengen von 0,5 bis 10, vorzugsweise 0,5 bis 8 Gew.-%, insbesondere 0,5 bis 6 Gew.-%.

Besonders wirksame Katalysatoren für die Ammonoxidation erhalten zusätzlich 0,01 bis 2,0 Gew.-% Cesium und/oder Rubidium, als Metall gerechnet. Günstig hat sich auch ein Gehalt von 0,05 bis 1,5 Gew.-% Wolfram, als Metall gerechnet, erwiesen.

Diese Mengenangaben beziehen sich jeweils auf die Gesamtmasse des Katalysators.

Außerdem kann die aktive Masse des Katalysators weitere Komponenten enthalten, z.B. Titan, Eisen, Cobalt, Nickel, Mangan oder Kupfer, wie sie im Schrifttum für die Ammonoxidation vorgeschlagen werden.

Zur Imprägnierung des Trägermaterials setzt man die aktiven Komponenten vorzugsweise in Form von wäßrigen Lösungen ihrer Salze ein, und zwar insbesondere von Salzen organischer Säuren, die sich bei der oxidativen Calcinierung rückstandsfrei zersetzen. Bevorzugt werden hierbei die Oxalate, besonders im Fall des Vanadiums, und die Tartrate, besonders im Fall des Antimons, wobei die Tartrate auch in Form gemischter Salze, z.B. mit Ammoniumionen, vorliegen können. Zur Herstellung solcher Lösungen kann man die Metalloxide in den Säuren auflösen. Wolfram wird bevorzugt in Form von Komplexverbindungen mit Weinsäure, Oxalsäure oder Citronensäure eingesetzt.

Die erfindungsgemäßen Katalysatoren eignen sich besonders für die Ammonoxidation in der Wirbelschicht. Die Technik dieser Verfahrensweise ist an sich bekannt, so daß nähere Ausführungen hierzu entbehrlich sind.

Vor allem dienen die Katalysatoren zur Herstellung ein- und mehrwertiger iso-und heteroaromatischer Nitrile aus den entsprechenden Alkylverbindungen, darunter insbesondere den Methylverbindungen.

Besondere Bedeutung hat die Ammonoxidation für die Herstellung von o-Phthalodinitril aus o-Xylol, von Isophthalodinitril aus m-Xylol, von Terephthalodinitril aus p-Xylol, von Benzonitril aus Toluol und von Nicotinsäurenitril aus β-Picolin.

Im Fall der Xylole verläuft die Ammonoxidation der ersten Methylgruppe schneller als die der zweiten, so daß sich auch leicht partielle Ammonoxidationsprodukte gewinnen lassen, z.B. p-Methylbenzonitril aus p-Xylol.

Die aromatischen Stammverbindungen können Substituenten tragen, welche sich unter den Bedingungen der Ammonoxidation inert verhalten, also z.B. Halogen oder die Trifluormethyl-, Nitro-, Amino- oder Cyano-Gruppe. Auch nicht-inerte Substituenten kommen in Betracht, wenn sie unter Bedingungen der Ammonoxidation in erwünschte Substituenten übergehen, wie beispielsweise die Aminomethyl- oder die Hydroxymethylgruppe.

Vorzugsweise nimmt man die Ausgangsverbindungen in einem Gasstrom aus Ammoniak und einem Sauerstoff enthaltenden Gas wie Luft auf, wobei ihre Konzentration zweckmäßigerweise auf 0,1 bis 25 Vol.%, vorzugsweise 0,1 bis 10 Vol-%, eingestellt wird.

Die erfindungsgemäßen Katalysatoren gestatten eine Katalysatorbelastung von 0,01 bis 1 kg der Ausgangsverbindung pro kg Katalysator und pro Stunde. Sie weisen den großen Vorteil auf, daß das Wirbelbett über lange Betriebszeiten konstant bleibt und daher keiner aufwendigen Regelung bedarf. Die Bildung von unerwünschten Nebenprodukten wird unterdrückt und dementsprechend erhöhen sich die Ausbeuten an den Verfahrensprodukten merklich.

Beispiel 1

Herstellung eines Trägerkatalysators

1000 g kugelförmiges Aluminiumoxid (Puralox[R] SCCa 150/120 Firma Condea-Chemie, Moers), welches einen Formfaktor von 0,85 und eine Schüttdichte von 0,8 kg/l hatte, wurde in einem Mischer mit 931,9 g einer wäßrigen Lösung imprägniert, die aus 351,4 g destilliertem Wasser, 150 g 25 %-iger $NH_3$-Lösung, 65,5 g Antimon-(III)-oxid, 56,2 g Vanadinsäure (90 % Vanadium-(V)-oxid), 6.5 g Cäsiumnitrat, 150 g Weinsäure und 152,3 g Oxalsäuredihydrat hergestellt worden war. Die Lösung wurde vollständig adsorbiert. Die erhaltene Masse wurde bei 120°C getrocknet und danach bei 580°C im Luftstrom calciniert.

Ammonoxidation von o-Xylol

In einem Wirbelbettversuchsreaktor von 6 cm Durchmesser und 120 cm Höhe wurde bei 470°C o-Xylol in einem Gasstrom bestehend aus 3 Vol-% o-Xylol, 12 Vol-% Sauerstoff und 85 Vol-% Ammoniak an diesem Katalysator (0,75 l) umgesetzt. Dabei wurde bei einem o-Xylol-Umsatz von 95,3 % eine o-Phthalodinitril-Ausbeute von 64,6 % (bez. auf o-Xylol) erzielt.

Vergleichsbeispiel

Herstellung eines Trägerkatalysators

Die Herstellung des Katalysators erfolgte auf analoge Weise, jedoch mit einem nadelförmigen Trägermaterial, das eine Schüttdichte von 0,5 kg/l aufwies, wie es in der EP-A 222 249 beschrieben ist.

Anmonoxidation von o-Xylol

Bei der Umsetzung in analoger Weise wurde bei einem o-Xylol-Umsatz von 95,8 % eine o-Phthalodinitril-Ausbeute von 62,8 % (bez. auf o-Xylol) erzielt.

Beispiel 2

1000 g kugelförmiges Aluminiumoxid (Puralox® SCC a 150/120 Firma Condea-Chemie, Moers), welches eine Schüttdichte von 0,8 kg/l hatte, wurde in einem Mischer mit 931,9 g einer wäßrigen Lösung imprägniert, die aus 351,4 g destilliertem Wasser, 150 g 25%iger Ammoniak-Lösung, 65,5 g Antimon-(III)-oxid, 56,2 g Vanadiumsäure (90 Gew.-% Vanadium(V)-Oxid), 12,5 g Cäsiumnitrat, 150 g Weinsäure und 152,3 g Oxalsäuredihydrat hergestellt worden war. Zusätzlich wurden 13,8 g Ammoniumparawolframat ($[NH_4]_{10}H_2W_{12}O_{42}$ x $5H_2O$), welches zuvor in 50 g Weinsäure und 200 ml Wasser gelöst wurde, in die Lösung zugegeben. Der imprägnierte Katalysatorvorläufer wird anschließend in einem Ofen bei 580°C zwei Stunden lang calciniert. Der Katalysator bestand nach chemischer Analyse zu 1,0 Gew.-% Wolfram.

Ammonoxidation von o-Xylol

In einem Wirbelschichtversuchsreaktor von 6 cm Durchmesser und 120 cm Höhe wurde bei 470°C o-Xylol in einem Gasstrom bestehend aus 3,2 Vol.-% o-Xylol (d.h. 128 g/h, 12 Vol.-% Sauerstoff und ca. 85 Vol.-% Ammoniak an diesem Katalysator (600 g) umgesetzt. Die zugespeiste Menge von o-Xylol wurde limitiert durch die Verkokung des Katalysators, was bei zu hoher o-Xylol-Dosierung zu starkem Umsatzrückgang führte. Dabei wurde bei einem o-Xylol-Umsatz von 95,7 % eine Ausbeute von 65,0 % Phthalodinitril (bezogen auf o-Xylol) erhalten. Bezogen auf 100 g Katalysator wurden somit stündlich 100,4 g Phthalodinitril erhalten.

**Patentansprüche**

1. Für die Ammonoxidation geeignete Trägerkatalysatoren aus

   a) einem Trägermaterial, das im wesentlichen aus Aluminiumoxid, Siliciumdioxid, Titandioxid und/oder Zirkoniumdioxid besteht und
   b) einer aktiven Masse, die als wesentliche Komponenten Vanadium und Antimon in oxidischer Form enthält,

   dadurch gekennzeichnet, daß das Trägermaterial kugelförmig oder annähernd kugelförmig ist und eine Schüttdichte von 0,6 bis 1,2 kg/l aufweist.

2. Trägerkatalysatoren nach Anspruch 1, enthaltend in der aktiven Masse b) zusätzlich Cesium und/oder Rubidium in oxidischer Form.

3. Trägerkatalysatoren nach Anspruch 1 oder 2, enthaltend in der aktiven Masse zusätzlich Wolfram in oxidischer Form.

4. Trägerkatalysatoren nach Anspruch 1 bis 3, enthaltend, bezogen auf die Masse des Trägerkatalysators, 0,5 bis 10 Gew.-% Vanadium und 0,5 bis 10 Gew.-% Antimon, jeweils als Metall berechnet.

5. Trägerkatalysatoren nach Anspruch 2, enthaltend, bezogen auf die Masse des Trägerkatalysators, 0,01 bis 2 Gew.-% Cesium und/oder Rubidium, als Metall berechnet.

6. Trägerkatalysator nach Anspruch 3, enthaltend, bezogen auf die Masse des Trägerkatalysators, 0,05 bis 1,5 Gew.-% Wolfram, als Metall berechnet.

7. Verfahren zur Herstellung der Trägerkatalysatoren gemäß den Ansprüchen 1 bis 6, dadurch gekennzeichnet, daß man ein kugelförmiges oder annähernd kugelförmiges Trägermaterial mit einer Lösung oder Suspension einer Vanadium-Verbindung sowie einer Antimon-Verbindung und gewünschtenfalls einer Wolfram-Verbindung und einer Cesium- und/oder Rubidium-Verbindung sowie weiterer Komponenten imprägniert, die erhaltene Mischung von überschüssiger Flüssigkeit abtrennt, trocknet und unter oxidierenden Bedingungen calciniert.

8. Verfahren zur Herstellung der Trägerkatalysatoren nach Anspruch 7, dadurch gekennzeichnet, daß man das Vanadium in Form eines Salzes der Oxalsäure einsetzt.

9. Verfahren zur Herstellung der Trägerkatalysatoren nach Anspruch 7, dadurch gekennzeichnet, daß man das Antimon in Form eines Salzes der Weinsäure einsetzt.

10. Verfahren zur Herstellung des Trägerkatalysators nach Anspruch 7, dadurch gekennzeichnet, daß man das Wolfram in Form einer Komplexverbindung mit Weinsäure, Oxalsäure oder Citronensäure einsetzt.

11. Verfahren zur Herstellung der Trägerkatalysatoren nach den Ansprüchen 7, dadurch gekennzeichnet, daß man die Calcinierung bei 400 bis 750°C vornimmt.

12. Verfahren zur Herstellung von ein- oder mehrwertigen isoaromatischen oder heteroaromatischen Nitrilen durch katalytische Ammonoxidation der entsprechenden iso- oder heteroaromatischen Alkylverbindungen mit einem Sauerstoff und Ammoniak enthaltenden Gas, dadurch gekennzeichnet, daß man hierzu einen Katalysator gemäß den Ansprüchen 1 bis 6 verwendet.

13. Verfahren nach Anspruch 12, dadurch gekennzeichnet, daß man die Umsetzung bei 300 bis 550°C vornimmt.

14. Verfahren nach Anspruch 12, dadurch gekennzeichnet, daß der Sauerstoffgehalt des zur Ammonoxidation eingesetzten Gases zwischen 0,1 und 25 Vol-% beträgt.

15. Verfahren nach den Ansprüchen 12, dadurch gekennzeichnet, daß man die Reaktion in einem Wirbelbett-Reaktor durchführt.

16. Verfahren nach den Ansprüchen 12, dadurch gekennzeichnet, daß man es auf die Ammonoxidation von Xylolen zu Methylbenzonitrilen oder Benzodinitrilen anwendet.

17. Verfahren nach den Ansprüchen 12, dadurch gekennzeichnet, daß man es auf die Ammonioxidation von o-Xylol zu Phthalodinitril anwendet.

## Claims

1. A supported catalyst which is suitable for ammonoxidation, comprising

   a) a support material which essentially comprises aluminum oxide, silicon dioxide, titanium dioxide and/or zirconium dioxide, and
   b) an active material which comprises, as essential components, vanadium and antimony in oxidic form,

   wherein the support material is spherical or approximately spherical and has a bulk density of from 0.6 to 1.2 kg/l.

2. A supported catalyst as claimed in claim 1, wherein the active material b) additionally comprises cesium and/or rubidium in oxidic form.

3. A supported catalyst as claimed in claim 1 or 2, wherein the active material additionally comprises tungsten in oxidic form.

4. A supported catalyst as claimed in claims 1 to 3, comprising, based on the weight of the supported catalyst, from 0.5 to 10% by weight of vanadium and from 0.5 to 10% by weight of antimony, in each case calculated as metal.

5. A supported catalyst as claimed in claim 2, comprising, based on the weight of the supported catalyst, from 0.01 to 2% by weight of cesium and/or rubidium, calculated as metal.

6. A supported catalyst as claimed in claim 3, comprising, based on the weight of the supported catalyst, from 0.05 to 1.5% by weight of tungsten, calculated as metal.

7. A process for the preparation of a supported catalyst as claimed in claims 1 to 6, wherein a spherical or approximately spherical support material is impregnated with a solution or suspension of a vanadium compound and of an antimony compound and, if desired, of a tungsten compound and of a cesium and/or rubidium compound and further components, and the resultant mixture is separated from excess liquid, dried and calcined under oxidizing conditions.

8. A process for the preparation of a supported catalyst as claimed in claim 7, wherein the vanadium is employed in the form of a salt of oxalic acid.

9. A process for the preparation of a supported catalyst as claimed in claim 7, wherein the antimony is employed in the form of a salt of tartaric acid.

10. A process for the preparation of a supported catalyst as claimed in claim 7, wherein the tungsten is employed in the form of a complex compound with tartaric acid, oxalic acid or citric acid.

11. A process for the preparation of a supported catalyst as claimed in claim 7, wherein the calcination is carried out at from 400 to 750°C.

12. A process for the preparation of isoaromatic or heteroaromatic mono- or polynitriles by catalytic ammonoxidation of the corresponding isoaromatic or heteroaromatic alkyl compounds by means of an oxygen- and ammonia-containing gas, wherein a catalyst as claimed in claims 1 to 6 is used.

13. A process as claimed in claim 12, wherein the reaction is carried out at from 300 to 550°C.

14. A process as claimed in claim 12, wherein the oxygen content of the gas employed for the ammonoxidation is from 0.1 to 25% by volume.

15. A process as claimed in claim 12, wherein the reaction is carried out in a fluidized-bed reactor.

16. A process as claimed in claim 12, which is used for the ammonoxidation of xylenes to methylbenzonitriles or benzodinitriles.

**17.** A process as claimed in claim 12, which is used for the ammonoxidation of o-xylene to phthalodinitrile.

**Revendications**

**1.** Catalyseurs sur supports adaptés pour l'ammoxydation comprenant

a) un matériau support constitué essentiellement d'oxyde d'aluminium, de dioxyde de silicium, de dioxyde de titane et/ou de dioxyde de zirconium et
b) une masse active contenant en tant que composants essentiels du vanadium et de l'antimoine sous forme oxydée,

caractérisés en ce que le matériau support a une forme sphérique ou quasi-sphérique et une densité apparente de 0,6-1,2 kg/l.

**2.** Catalyseurs sur support selon la revendication 1, contenant en outre dans la masse active b) du césium et/ou du rubidium sous forme oxydée.

**3.** Catalyseurs sur support selon la revendication 1 ou 2, contenant en outre dans la masse active du tungstène sous forme oxydée.

**4.** Catalyseurs sur support selon l'une quelconque des revendications 1 à 3, contenant, par rapport à la masse du catalyseur sur support, 0,5-10% en poids de vanadium et 0,5-10% en poids d'antimoine, comptés à chaque fois en tant que métal.

**5.** Catalyseurs sur support selon la revendication 2, contenant, par rapport à la masse du catalyseur sur support, 0,01-2% en poids de césium et/ou de rubidium, comptés en tant que métal.

**6.** Catalyseurs sur support selon la revendication 3, contenant, par rapport à la masse du catalyseur sur support, 0,05-1,5% en poids de tungstène, compté en tant que métal.

**7.** Procédé de préparation de catalyseurs sur support selon l'une quelconque des revendications 1 à 6, caractérisé en ce que l'on imprègne un matériau support ayant une forme sphérique ou quasisphérique avec une solution ou une suspension d'un composé du vanadium, d'un composé de l'antimoine et éventuellement d'un composé du tungstène et d'un composé du césium et/ou du rubidium ainsi que d'autres composants, on sépare le mélange obtenu du liquide en excès, on sèche et on calcine dans des conditions oxydantes.

**8.** Procédé de préparation de catalyseurs sur support selon la revendication 7, caractérisé en ce que l'on utilise le vanadium sous la forme d'un sel de l'acide oxalique.

**9.** Procédé de préparation de catalyseurs sur support selon la revendication 7, caractérisé en ce que l'on utilise l'antimoine sous la forme d'un sel de l'acide tartrique.

**10.** Procédé de préparation de catalyseurs sur support selon la revendication 7, caractérisé en ce que l'on utilise le tungstène sous la forme d'un complexe avec l'acide tartrique, l'acide oxalique ou l'acide citrique.

**11.** Procédé de préparation de catalyseurs sur support selon la revendication 7, caractérisé en ce que l'on mène la calcination à 400-750°C.

**12.** Procédé de préparation de nitriles isoaromatiques ou hétéroaromatiques mono ou polyfonctionnels par ammoxydation catalytique des composés alkylés iso- ou hétéroaromatiques correspondants avec un gaz contenant de l'oxygène et de l'ammoniac, caractérisé en ce que l'on utilise à cet effet un catalyseur selon l'une quelconque des revendications 1 à 6.

**13.** Procédé selon la revendication 12, caractérisé en ce que l'on mène la réaction à 300-550°C.

**14.** Procédé selon la revendication 12, caractérisé en ce que la teneur en oxygène du gaz utilisé pour l'ammoxydation s'élève à 0,1-25% en volume.

**15.** Procédé selon la revendication 12, caractérisé en ce que l'on mène la réaction dans un réacteur à lit fluidisé.

**16.** Procédé selon la revendication 12, caractérisé en ce qu'on l'applique à l'ammoxydation de xylols en méthylbenzonitriles ou en benzodinitriles.

**17.** Procédé selon la revendication 12, caractérisé en ce qu'on l'applique à l'ammoxydation du o-xylol en phtalodinitrile.